# EUROPÄISCHE PATENTSCHRIFT

(11) **EP 1 805 221 B1**
(45) Veröffentlichungstag und Bekanntmachung des Hinweises auf die Patenterteilung: **11.08.2010**
(21) Anmeldenummer: 05808248.8
(22) Anmeldetag: 13.10.2005
(51) Int. Cl.: C07K 16/28, C12N 5/20, G01N 33/68, G01N 33/577, A61K 39/395

(54) **MONOKLONALER ANTIKÖRPER GEGEN HUMANEN FRIZZLED-4-REZEPTOR**
MONOCLONAL ANTIBODY AGAINST HUMAN FRIZZLED-4 RECEPTOR
ANTICORPS MONOCLONAUX CONTRE LE RECEPTEUR FRIZZLED-4 HUMAIN

(30) Priorität: 13.10.2004 DE 102004050620
(43) Veröffentlichungstag der Anmeldung: 11.07.2007
(73) Patentinhaber: Eberhard-Karls-Universität Tübingen Universitätsklinikum, 72076 Tübingen (DE)
(72) Erfinder: BÜHRING, Hans-Jörg, 72076 Tübingen (DE); HOJAK, Sigrid, 10437 Berlin (DE); ALBERT, Ingrid, 72072 Tübingen (DE)
(74) Vertreter: Findeisen, Marco
(86) Internationale Anmeldenummer: PCT/EP2005/011035
(87) Internationale Veröffentlichungsnummer: WO 2006/040163

(56) Entgegenhaltungen:
- WO-A-99/26960
- WO-A-02/088081
- WO-A-03/005034
- WO-A-2004/020668

## Beschreibung

Die vorliegende Erfindung betrifft einen monoklonalen Antikörper oder Fragment davon, der bzw. das spezifisch oder/und selektiv an humanen Frizzled-4 Rezeptor (FZD-4 Rereptor) bindet.

Ferner betrifft sie eine Hybridomzelle, die den erfindungsgemäßen Antikörper produziert. Weiterer Gegenstand der vorliegenden Erfindung ist ein Verfahren zur Detektion oder/und Isolierung von humanen Frizzled-4 Rezeptor oder Homologen oder Fragmenten davon in einer biologischen Probe sowie ein Verfahren zur Identifizierung oder/und Isolierung von biologischem Material, das humanen Frizzled-4 Rezeptor oder Homologe oder Fragmente davon aufweist bzw. exprimiert. Ferner betrifft die Erfindung die Verwendung des erfindungsgemäßen Antikörpers oder Fragmenten davon zur spezifischen oder/und selektiven Detektion oder/und Isolierung von humanen Frizzled-4 Rezeptor, eine Zusammensetzung, die den erfindungsgemäßen monoklonalen Antikörper oder Fragmente davon aufweist sowie ein Kit, das den erfindungsgemäßen monoklonalen Antikörper oder Fragmente hiervon aufweist.

Frizzled(FZD)-Rezeptoren sind Proteine vom 7-Transmembrantyp, die ein Sequenzmotiv mit sieben α-helikalen Domänen aufweisen, über die eine Verankerung in der Membran erfolgt. Dieses Motiv ist typisch für die sog. G-Protein-gekoppelten Rezeptoren (GPCRs). Manche Autoren zählen deshalb die Frizzled-Rezeptoren auch zur Superfamilie der GPCRs; vgl. bspw. Malbon C. C. (2004), "Frizzleds: new members of the superfamily of G-protein-coupled receptors", Front. Biosci., Vol. 9, Seiten 1048 bis 1058. Die Frizzled-Rezeptoren weisen ferner eine cysteinreiche Domäne (CRD) an ihrem aminoterminalen Ende auf.

Frizzled-Rezeptoren sind wichtige Komponenten der sog. Wnt(Wingless-Type)-Signalkette, die sowohl bei Wirbeltieren als auch wirbellosen Tieren konserviert ist. Die wichtigsten Liganden der Frizzled-Rezeptoren sind die Wnt-Proteine, die über die Bindung an den extrazellulären Teil des Frizzled-Rezeptors intrazelluläre Ereignisse induzieren. An der Aufnahme und Weiterleitung von Wnt-Signalen sind neben den FZD-Rezeptoren Mitglieder einer weiteren Klasse von Zelloberflächenrezeptoren aus der Familie der LDL-Rezeptor-verwandten Proteine (LDL-receptorrelated proteins, LRP) beteiligt.

Die intrazelluläre Signalweiterleitung kann im Wesentlichen über zwei unterschiedliche Wege erfolgen. Liganden der Wnt5A-Klasse stimulieren die Signaltransduktion über die Pertussis-Toxin-sensitive Gruppe der heterotrimären G-Proteine über den Anstieg der intrazellulären Ca²⁺-Konzentration sowie über die RhoA/c-jun-N-terminale Kinase-Signalkastrade (sog. Wnt-Ca²⁺-Weg). Bei Liganden der Wntl-Klasse dagegen erfolgt die Signalweiterleitung über ein weiteres wichtiges intrazelluläres Signalprotein, β-Catenin (sog. Wnt/β-Catenin-Weg). β-Catenin ist ein Transkriptionsaktivator. In Abwesenheit eines Wnt-Signales ist ein Teil des intrazellulären β-Catenins an den cytosolischen Schwanz des sog. Cadherin-Proteins gebunden und ein weiterer Teil des cytosolischen β-Catenins befindet sich in einem Komplex aus den Proteinen Axin, APC (Adenomatous Polyposis coli) und GSK-3β (Glycogen-Syntase Kinase 3). In diesem sog. Degradationskomplex wird β-Catenin durch GSK-3β phosphoryliert und triggert damit seine eigene Ubiquitinylierung und damit Degradierung in Proteasomen. β-Catenin ist demnach in Abwesenheit von Wnt instabil und kann seine Funktion als Transkriptionsaktivator nicht ausüben. In Abwesenheit von Wnt sind deshalb verschiedene Wnt-regulierte Gene inaktiviert, was durch ein Corepressorprotein mit der Bezeichnung Groucho sichergestellt wird, das zusammen mit den transkriptionsregulierenden Proteinen LEF-1/TCF an die DNA im Promotorbereich dieser Gene gebunden ist.

Bei der Bindung von Wnt an den Frizzled-Rezeptor und seinen Rezeptor LRP kommt es über einen bislang unbekannten Mechanismus zur Aktivierung des intrazellulären Proteins Dishevelled. Aktiviertes Dishevelled induziert über einen ebenfalls bislang unbekannten Mechanismus die Inaktivierung von GSK-3β in dem Degradationskomplex. Daraus resultierend wird die Phosphorylierung und Degradation von β-Catenin inhibiert. β-Catenin ist dann stabil und akkumuliert im Cytoplasma und im Nucleus. Im Nucleus bindet β-Catenin an LEF-1/TCF (Lymphoid Enhancer Factor-1/T cell-specific Factor), setzt den Faktor Groucho frei und stimuliert somit die Transkription von Wnt-regulierten Genen. Ein Überblick über die Wnt-Signalkette findet sich bspw. in Huelsken J. und Behrens J. (2002), "The Wnt signalling pathway", J. Cell Sci., Vol. 115, Seiten 3977 bis 3978.

Neben Wnt wurde kürzlich ein weiterer Ligand für Frizzled-Rezeptoren gefunden, der den Namen Norrin trägt; vgl. Niehrs C. (2004), "Norrin and Frizzled: A New Vein for the Eye", Developmental Cell, Vol. 6, Seiten 1 bis 20.

Frizzled-Rezeptoren spielen eine wichtige Rolle in der Differenzierung und Organogenese embryonaler Gewebe, bei der Oogenese und Keimblattentwicklung sowie bei der Selbsterneuerung von Stammzellen. Folglich ist eine Vielzahl von Arbeitsgruppen aus dem Bereich der Grundlagenforschung mit der näheren Charakterisierung dieser Rezeptoren beschäftigt.

Frizzled-Rezeptoren scheinen jedoch auch bei einer Vielzahl von Krankheiten eine wichtige Rolle zu spielen. So kommt diesen Rezeptoren eine Schlüsselfunktion bei der Karzinogenese zu; vgl. Holcombe R. F. et al. (2002), "Expression of Wnt ligands and Frizzled receptors in colonic mucosa and in colon carcinoma", J. Clin. Pathol. Vol. 55(4), Seiten 220 bis 226, sowie Karim R. et al. (2004), "The significance of the Wnt pathway in the pathology of human cancers", Pathology 36(2), Seiten 120 bis 128.

Ferner wurde ein Zusammenhang mit der sog. Norrie-Krankheit festgestellt, einer X-chromosomal-rezessiven Erkrankung, die durch degenerative Veränderungen in der Netzhaut gekennzeichnet ist; vgl. Xu et al. (2004), "Vascular development in the retina and inner ear: control by Norrin and Frizzled4, a high-affinity ligand-receptor pair", Cell, Vol. 116(6), Seiten 883 bis 895; eine Involvierung von Frizzled-Rezeptoren in das Krankheitsbild der familiären exsudativen Vitreoretinopathie, einer progredienten Netzhauterkrankung; vgl. Robitaille et al. (2002), "Mutant frizzled-4 disrupts retinal angiogenesis in familial exudative vitreoretinopathy", Nat. Genet., Vol. 32(2), Seiten 326 bis 330.

Ferner sind Frizzled-Rezeptoren an dem Krankheitsbild der rheumatoiden Arthritis beteiligt; vgl. Sen et al. (2001), "Blockade of Wnt-5A/frizzled 5 signaling inhibits rheumatoid synoviocyte activation", Arthritis Rheum., Vol. 44(4), Seiten 772 bis 781. Darüber hinaus findet man in der Literatur Frizzled-Rezeptoren im Zusammenhang mit einer Vielzahl von weiteren Krankheitsbildern beschrieben.

Vor diesem Hintergrund besteht sowohl für die Forschung als auch die Diagnostik und Pharmazeutik großer Bedarf an Antikörpern, mittels denen selektiv Frizzled-Rezeptoren erkannt bzw. identifiziert werden können.

Im Stand der Technik sind umfangreich polyklonale Antikörper gegen verschiedene Frizzled-Rezeptoren beschrieben, wie bspw. gegen humanes FZD-5 (Sen et al., a.a.O.), gegen Maus-FZD-9 (Van Raay et al. (2001), "Frizzled 9 is expressed in neural precursor cells in the developing neural tube", Dev. Genes Evol., Vol. 211(8-9), Seiten 453 bis 457), oder gegen humanes FZD-1 und FZD-2 (Holcombe R. F. et al., a.a.O.).

Der Nachteil derartiger polyklonaler Antikörper liegt darin, dass diese nicht unbegrenzt zur Verfügung stehen und nach dem Verbrauch nicht mehr identisch nachproduziert werden können. Ferner handelt es sich bei polyklonalen Antikörpern naturgemäß um ein Gemisch von unterschiedlichen Antikörpern, die jeweils gegen verschiedene antigene Strukturen des jeweiligen Epitops gerichtet sind. Folglich ist es mittels polyklonaler Antikörper nicht möglich, reproduzierbar immer wieder die gleiche antigene Struktur zu identifizieren.

Ein weiterer Nachteil von polyklonalen Antikörpern liegt darin, dass diese zum Teil sehr hohe Kreuzreaktivitäten mit weiteren nicht-interessierenden Proteinen zeigen, was deren Einsatz im Immunoblot oder auch in der Immunpräzipitation stark einschränkt und eine diagnostische oder therapeutische Verwendung dieses Gemisches unmöglich macht.

Es besteht deshalb verstärkter Bedarf an der Bereitstellung von monoklonalen Antikörpern, die gegen Frizzled-Rezeptoren, insbesondere gegen die humanen Varianten, gerichtet sind. Es hat sich jedoch gezeigt, dass die Herstellung derartiger monoklonaler Antikörper besonders schwierig ist, da Frizzled-Rezeptoren im Tierreich hinsichtlich ihrer Aminosäuresequenz und damit ihrer dreidimensionalen Struktur sehr stark konserviert sind. Es wird deshalb angenommen, dass es nahezu unmöglich ist, monoklonale Antikörper herzustellen, über die eine speziesspezifische Abgrenzung der Frizzled-Rezeptoren (z.B. Frizzled-4 Rezeptoren) möglich ist.

Die Firma R&D Systems Inc., Minneapolis, MN, USA, bietet monoklonale Antikörper gegen die Maus-Variante von Frizzled-1 (Klon 162531), -3 (Klon 169310), -4 (Klon 145901) und -7 (Klon 151143) an. Der Hersteller wirbt damit, dass mit diesen Antikörpern auch die jeweiligen humanen Varianten der Frizzled-Rezeptoren detektiert werden könnten. Dabei hat sich in der Fachwelt jedoch gezeigt, wie dies von den Erfindern auch verifiziert wurde, dass eine zufriedenstellende Detektion der humanen Varianten mittels dieser Antikörper nicht möglich ist. Vielmehr erfolgt die Bindung dieser Antikörper an humane Rezeptoren der Frizzled-Familie auf unspezifische und unselektive Art und Weise, so dass lediglich eine gewisse Kreuzreaktivität mit den humanen Varianten verbunden mit einer hohen Affinität gegenüber weiteren nicht gewünschten Strukturen festzustellen ist. Der Einsatz dieser monoklonalen Antikörper zur selektiven oder/und spezifischen Identifizierung von humanen Frizzled-Rezeptoren ist deshalb nicht möglich.

Aufgabe der vorliegenden Erfindung ist es deshalb, einen monoklonalen Antikörper bereitzustellen, der spezifisch oder/und selektiv an humane Rezeptoren der Frizzled-Familie bindet.

Den Erfindern ist es nun zum ersten Mal gelungen, diese Aufgabe zu lösen. Vorliegend wird ein solcher monoklonaler Antikörper bereitgestellt. Konkret konnten die Erfinder einen monoklonalen Antikörper herstellen, der hochspezifisch und selektiv an die humane Variante von FZD-4 bindet. Dieser Antikörper wird von Hybridomzellen produziert, die in Kultur bei der Deutschen Sammlung für Mikroorganismen und Zellkulturen (DSMZ), Mascheroder Weg 1b, 38124 Braunschweig, unter der Nummer DSM ACC 2667 (FZD-4) nach dem Budapester Vertrag am 15. Juli 2004 hinterlegt wurden.

Dieser monoklonale Antikörper hat überraschende und nicht vorhersehbare Eigenschaften. So bindet er hochselektiv und spezifisch an die humane Variante des Frizzled-4 Rezeptors und zeigt folglich lediglich äußerst geringe Kreuzreaktivitäten mit den Varianten anderer Spezies. Dies war auf Grund der hohen Konservierung des Frizzled-4 Rezeptors im Tierreich nicht zu erwarten. Es wurde vielmehr bislang angenommen, dass auf Grund der strukturellen Ähnlichkeiten der Frizzled-4 Rezeptoren verstärkt Kreuzreaktivitäten mit Varianten anderer Spezies unvermeidbar sind.

Erfindungsgemäß bedeutet eine spezifische oder/und selektive Bindung an humanen FZD-4-Rezeptor, dass der monoklonale Antikörper konkret gegen humanen FZD-4-Rezeptor etabliert wurde, nicht aber gegenüber einer Variante einer anderen Spezies, wie bspw. der Maus, der dann lediglich kreuzreaktiv mit der humanen Variante wäre.

Im Rahmen der vorliegenden Erfindung kann statt des erfindungsgemäßen Antikörpers auch ein Fragment desselben verwendet werden. Unter einem solchen Fragment wird dabei jeder Abschnitt des Antikörpers verstanden, der die Antigenbindungsfunktion des Antikörpers beibehält. Solche Fragmente sind bspw. F_{ab}, F_{(ab')2}, Fᵥ und andere Fragmente, wie z.B. CDR("complementary determining region", hypervariable Region)-Fragmente. Die genannten Fragmente weisen ebenfalls die Bindungsspezifität des Antikörpers auf und können auch bspw. mit bekannten Verfahren als rekombinante Proteine hergestellt werden.

Die besondere Leistung der Erfinder liegt darin, dass nun mit dem hinterlegten Antikörper nicht nur ein hochselektives Tool zur Verwendung in Forschung und Medizin bereitgestellt wird, sondern dass mittels dieses Antikörpers auch weitere monoklonale Antikörper erzeugt werden können, die an das gleiche Antigen binden. Die Erfinder haben dadurch, dass dieser unerwarteterweise hochselektive Antikörper gegen die humane Variante des FZD-4-Rezeptors hergestellt werden konnte, eine wichtige Hürde genommen und so den Weg für die Entwicklung vergleichbarer monoklonaler Antikörper geebnet. So ist es nämlich möglich, das Antigen, das von den hinterlegten Antikörpern erkannt wird, zu isolieren und zur Immunisierung bspw. von Mäusen zu verwenden. Die sich daran anschließende Herstellung von monoklonalen Antikörpern ist im Stand der Technik grundsätzlich beschrieben; vgl. Köhler G. und Milstein C. (1975), "Continuous cultures of fused cells secreting antibody of predefined specificity", Nature, Vol. 256(5517), Seiten 495 bis 497. Danach werden den Tieren nach der Immunisierung mit dem isolierten Antigen die Antikörper produzierenden Zellen entnommen und mit einer immortalisierten Zelllinie fusioniert. Aus den resultierenden Hybridomzelllinien wird eine solche ausgewählt, die Antikörper, und dies in einer unbegrenzten Menge, gegen das Antigen produziert, das für die Immunisierung eingesetzt wurde.

Gegenstand der vorliegenden Erfindung sind auch die konkret bei der DSMZ hinterlegten Antikörper.

Der mit der Nr. DSM ACC 2667 hinterlegte monoklonale Antikörper weist den Isotyp Maus IgG1 auf, der selektiv humanes FZD-4 erkennt, nicht jedoch andere Rezeptoren der Frizzled-Familie, bspw. FZD-1, -5, -6, -7, -9 oder -10.

Mit den erfindungsgemäßen Antikörpern können die Phänotypen von hämatopoetischen, mesenchymalen und neuronalen Stamm-/Progenitorzellpopulationen (HSC, MSC, NPC) hinsichtlich ihrer FZD-Expression analysiert werden. So haben die Erfinder bereits eine Vielzahl von verschiedenen Zelltypen untersucht, wie Tumorzellen verschiedenster Art, einschließlich Leukämiezellen, Primärzellen und kultivierte Primärzellen. Dabei wurde überraschenderweise festgestellt, dass verschiedenste Zellen ein charakteristisches FZD-Expressionsspektrum zeigen, so dass die erfindungsgemäßen Antikörper deshalb zur Isolierung dieser Zellen aus einem heterogenen Zellgemisch verwendet werden können. So lassen sich bspw. mit dem hinterlegten monoklonalen Antikörper gegen FZD-4 auf vorteilhafte Art und Weise FZD-4-positive Zellen, wie neuronale Progenitorzellen erkennen und isolieren.

Ein weiterer Gegenstand der vorliegenden Erfindung betrifft einen vorstehend beschriebenen monoklonalen Antikörper oder ein Fragment hiervon, an den bzw. an das ein Marker oder/und ein therapeutischer Wirkstoff gekoppelt ist.

Erfindungsgemäß wird unter einem Marker eine jegliche Verbindung verstanden, mittels der eine Lokalisierung und Identifizierung der Antikörper und damit der FZD-Rezeptoren *in vitro* oder *in vivo* oder auch *in situ* möglich ist. Hierzu zählen Farbindikatoren, wie Farbstoffe, mit fluoreszierenden, phosphoreszierenden oder chemilumineszierenden Eigenschaften, AMPPD, CSPD, radioaktive Indikatoren, wie ³²P, ³⁵S, ¹²⁵I, ¹³¹I, ¹⁴C, ³H, nicht radioaktive Indikatoren, wie Biotin oder Digoxigenin, alkalische Phosphatase oder Meerrettich-Peroxidase. Der Nachweis solcher markierter Antikörper erfolgt dann über im Stand der Technik bekannte bildgebende Verfahren, wie Autoradiographie, Blotting-, Hybridisierungs- oder Mikroskopietechniken.

Als therapeutischer Wirkstoff kommt ein jeder Wirkstoff in Frage, der im Organismus eine spezifische Reaktion induziert, wie bspw. ein Arzneimittel oder auch ein Toxin. Auf diese Art und Weise kann ein Wirkstoff zielgerichtet in die Nähe eines FZD-Rezeptors gebracht werden und bspw. direkt auf die diesen Rezeptor exprimierende Zelle wirken. Damit wird vermieden, dass ein solcher Wirkstoff in Bereiche eines Organismus bzw. Organs gelangt, in denen eine Wirkung nicht erwünscht ist.

Ein weiterer Gegenstand der vorliegenden Erfindung betrifft eine Hybridomzelle, die den bzw. die vorstehend erläuterten Antikörper produziert.

Mithilfe dieser Hybridomzelle kann, wenn diese in Kultur genommen wird, ein entsprechender Antikörper, der von dieser produziert wird, in unbegrenzter Menge erhalten und zur Verfügung gestellt werden. Im Rahmen von wissenschaftlichen oder diagnostischen Anwendungen ist damit eine Reproduzierbarkeit der Ergebnisse gewährleistet. Dies ist Voraussetzung für die Zulassung als Arzneimittel.

Ein weiterer Gegenstand der vorliegenden Erfindung betrifft ein Verfahren zur Detektion oder/und Isolierung von humanen Rezeptoren der FZD-Familie oder Homologen oder Fragmenten davon in einer biologischen Probe, das folgende Schritte aufweist: (1) Bereitstellung der biologischen Probe; (2) In-Kontakt-bringen der biologischen Probe mit einem Antikörper; (3) Feststellung, ob eine spezifische oder/und selektive Bindung an den Antikörper stattgefunden hat, und (4.1) Korrelation einer positiven Feststellung in Schritt (3) mit der Detektion eines Rezeptors aus der FZD-Familie, oder/und (4.2) Isolierung des Antigens bei einer positiven Feststellung in Schritt (3), wobei als Antikörper der vorstehend beschriebene Antikörper oder das Fragment davon verwendet wird.

Mit diesem Verfahren lassen sich humane Rezeptoren der FZD-Familie zielgerichtet nachweisen. Hierzu werden die Schritte (1), (2), (3) und (4.1) durchgeführt. Bei einer zusätzlichen Durchführung von Schritt (4.2) nach oder anstelle von (4.1) kann jedoch auch das Antigen, bspw. der Rezeptor selbst, isoliert werden und zur oben angesprochenen Herstellung weiterer vergleichbarer monoklonaler Antikörper verwendet werden.

Mit diesem Verfahren lassen sich auch bislang unbekannte Homologe der humanen FZD-Rezeptoren auffinden, worunter erfindungsgemäß Komponenten mit äußerst ähnlichen Strukturen wie denen der jeweiligen FZD-Rezeptoren verstanden werden, die bislang u.U. nicht beschrieben wurden.

Ferner können mit diesem Verfahren große Mengen an FZD-Rezeptoren für eine anschließende Kristallisierung und Strukturaufklärung gewonnen werden. Darüber hinaus besteht die Möglichkeit, die FZD-Rezeptoren mit ihren natürlichen Liganden oder anderen mit diesen wechselwirkenden Faktoren oder Molekülen in großen Mengen zu isolieren, eine Co-Kristallisierung der Komplexe durchzuführen, um so Daten über die dreidimensionale Struktur dieser Komplexe zu erhalten. Damit wird beispielsweise die Grundlage für die Entwicklung von spezifischen mit FZD-Rezeptoren wechselwirkenden Substanzen (drug design) geschaffen, die die korrekte dreidimensionale Gestalt haben.

Bei der biologischen Probe kann es sich um Zellen, Zellkulturen, Zellfragmente, Gewebe, Proteinkonzentrate oder sonstige Lösungen oder Suspensionen handeln, die biologisches Material enthalten, oder aber die auf das Vorhandensein von biologischem Material untersucht werden sollen.

Das Verfahren kann in einem geeigneten Puffersystem durchgeführt werden, bei dem bspw. Tris-, Good- oder Phosphatpuffer eingesetzt werden. Das In-Kontakt-bringen bzw. die Inkubation in Schritt (2) erfolgt dabei ggf. unter Schütteln, nach allgemein bekannten biochemischen Techniken.

Die Feststellung in Schritt (3) erfolgt über im Stand der Technik allgemein bekannte Verfahren wie Immunblot, Immunpräzipitation oder sonstige Immuntechniken.

Mithilfe eines solchen Verfahrens können auch im Rahmen der Grundlagenforschung die humanen Varianten der FZD-Rezeptoren hinsichtlich ihrer bislang weitgehend unbekannten biochemischen Funktionsweise näher charakterisiert werden. So können die Rezeptoren oder Fragmente hiervon nach der Isolierung bspw. kristallisiert und in ihrer dreidimensionalen Struktur aufgeklärt werden. In diesem Zusammenhang lassen sich dann auch bspw. mit der Methode des "rational drug design" Wirkstoffe gezielt konstruieren, die selektiv an die FZD-Rezeptoren binden. Mit dem erfindungsgemäßen Verfahren wird demnach eine Methode bereitgestellt, die einen ersten Schritt für ein besseres Verständnis der FZD-Rezeptoren als auch für die Entwicklung eines zielgerichteten Wirkstoffes darstellt.

Ein weiterer Gegenstand der vorliegenden Erfindung betrifft ein Verfahren zur Identifizierung von biologischem Material hinsichtlich des Vorhandenseins oder/und der Expression von Rezeptoren der FZD-Familie oder Homologen oder Fragmenten davon und ggf. zur Isolierung eines solchen Materials, das folgende Schritte aufweist: (1) Bereitstellung des biologischen Materials; (2) In-Kontakt-bringen des biologischen Materials mit einem Antikörper; (3) Feststellung, ob eine spezifische oder/und selektive Bindung an den Antikörper stattgefunden hat, und (4.1) Korrelation einer positiven Feststellung in Schritt (3) mit der Identifizierung des Vorhandenseins oder der Expression von Rezeptoren der FZD-Familie oder Homologen oder Fragmenten davon in bzw. auf dem biologischen Material, und ggf.

(4.2) Isolierung des Bindepartners bei einer positiven Feststellung in Schritt (3), wobei als Antikörper der vorstehend beschriebene Antikörper oder das Fragment davon verwendet wird.

Dieses Verfahren ist gegenüber dem vorstehend beschriebenen Verfahren derart modifiziert, dass biologisches Material, bspw. Zellen wie Stammzellen, Zellfragmente etc., hinsichtlich ihres Phänotyps in Bezug auf die FZD-Expression identifiziert und isoliert werden kann.

Vor diesem Hintergrund ist Gegenstand der vorliegenden Erfindung auch die Verwendung des erfindungsgemäßen Antikörpers zur spezifischen oder/und selektiven Detektion oder/und Isolierung von Rezeptoren der FZD-Familie, vorzugsweise zur Analyse der Karzinogenese oder/und der Differenzierung und Organogenese embryonaler Gewebe oder/und der Oogenese und Keimblattentwicklung oder/und der Selbsterneuerung von Stammzellen.

Ein weiterer Gegenstand betrifft eine Zusammensetzung, vorzugsweise eine pharmazeutische Zusammensetzung, die den erfindungsgemäßen Antikörper oder Fragmente davon aufweist, sowie vorzugsweise einen pharmazeutisch akzeptablen Träger und ggf. weitere Zusätze.

Pharmazeutisch akzeptable Träger sind im Stand der Technik allgemein bekannt und werden bspw. in der Abhandlung von Kibbe A. (2000), "Handbook of Pharmaceutical Excipients", Third Edition, American Pharmaceutical Association and Pharmaceutical Press, beschrieben. Zusätze umfassen erfindungsgemäß jedwede Verbindung oder Zusammensetzung, die für eine therapeutische Verwendung vorteilhaft ist, worunter Salze, Bindemittel, aber auch weitere Wirkstoffe fallen.

Ein anderer Gegenstand betrifft ein Kit, das den erfindungsgemäßen monoklonalen Antikörper oder Fragmente davon aufweist.

Die Bereitstellung des erfindungsgemäßen Antikörpers in einem solchen Kit, ggf. zusammen mit weiteren Reagenzien, Puffern und einer Anleitung, wie der Antikörper zu verwenden ist, hat den Vorteil, dass die Anwendung deutlich vereinfacht wird und bspw. in Kliniken oder Großlabors, in denen z.T. auch angelerntes Personal arbeitet, Sicherheit geschaffen wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun anhand von Ausführungsbeispielen näher erläutert, die rein beispielhaften Charakter haben und die Tragweite der vorliegenden Erfindung in keiner Weise einschränken. Darin wird auf die beiliegende Figur Bezug genommen, in der zu sehen ist:

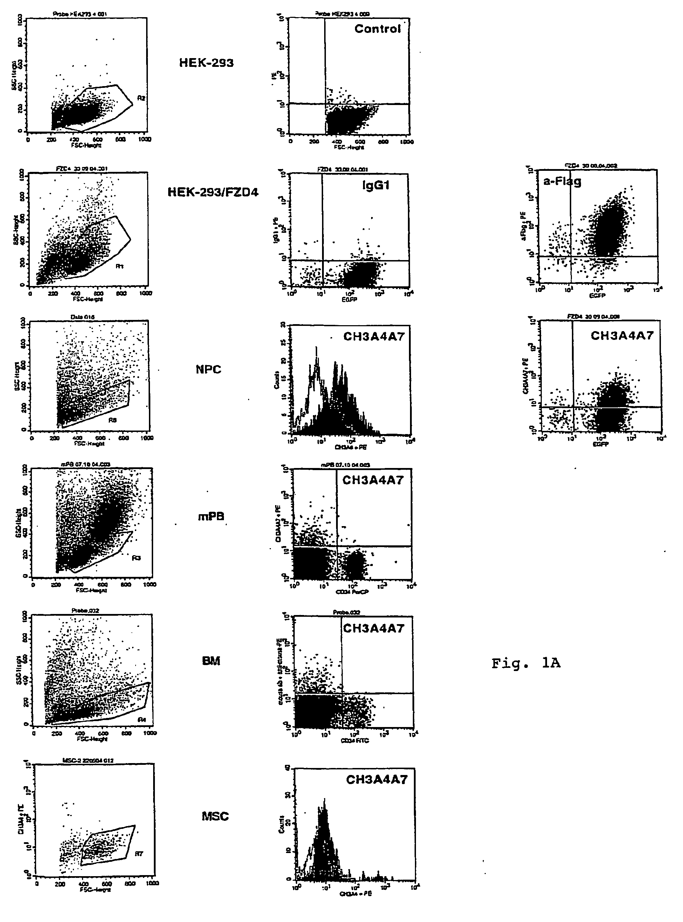

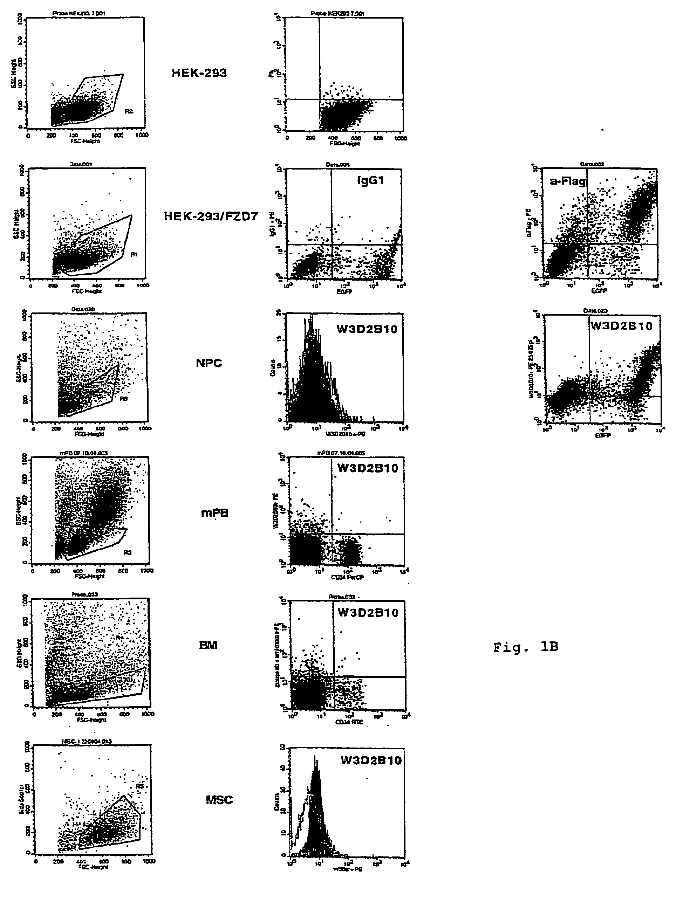

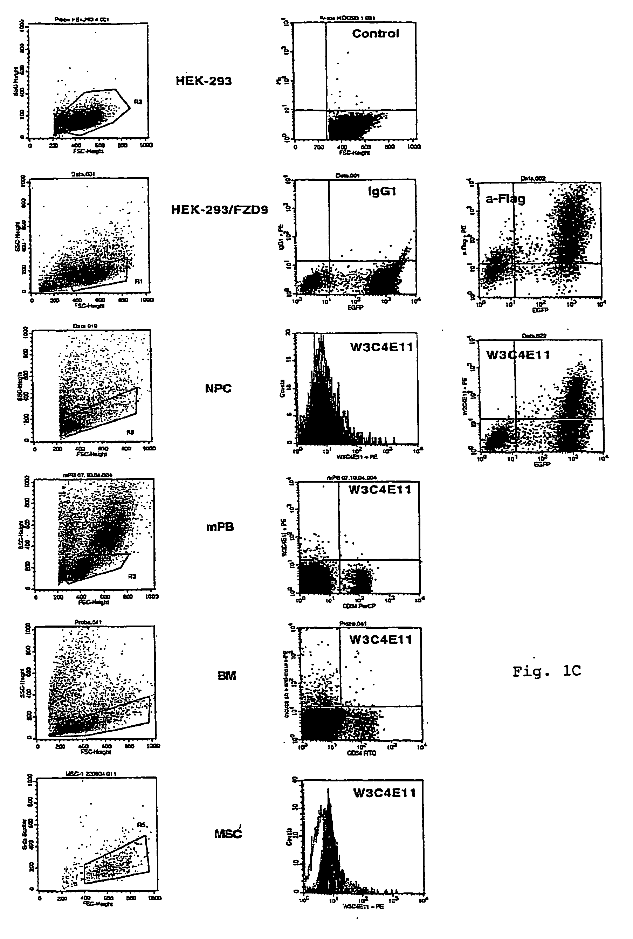

- Fig. 1: FACS-Histogramme zur Reaktivität der Antikörper CH3A4A7 (A), W3D2B10 (B) und W3C4E11 (C).

### Ausführungsbeispiel

### Herstellung von Transfektanten

Es wurden cDNAs hergestellt, die für FZD-4, -7 und -9 kodieren. Diese wurden in den Klonierungsvektor plRES-EGFP (Becton and Dickinson Biosciences, Franklin Lakes, NJ, USA) nach den Angaben des Herstellers kloniert. Am N-terminalen Ende wurde ein Flag-TAG inseriert, um die Membranständigkeit der FZD-Rezeptoren nachzuweisen. Mit diesen plRES-EGFP-Konstrukten wurden HEK-293-Zellen (DSMZ Nr. ACC 305) transfiziert.

Die so transfizierten HEK-293-Zellen wurden gelelektrophoretisch aufgetrennt, die Proteine auf eine Nitrozellulosemembran transferiert. Anschließend wurden Westernblots mit Antiseren enthaltend polyklonale Antikörper gegen FZD-4, -7 und -9 durchgeführt (Acris Antibody GmbH, 32120 Hiddenhausen, Deutschland). Dabei zeigte sich, dass sämtliche Transfektanten die gewünschten FZD-Konstrukte extrimieren (Daten nicht gezeigt).

Eine Woche nach der Transfektion wurden die Zellen mit einem anti-Flag-Antikörper und einem PE-konjugierten anti-Maus-Antikörper markiert und die EGFP- und Flag-positiven Zellen in einem FACS-Sortiergerät über mehrere Runden sortiert, bis eine Transfektante generiert wurde, die stark positiv für EGFP und Flag war. Nach drei bis vier Sortierzyklen konnte in der Regel eine stabile Transfektante hergestellt werden.

### Immunisierung

Die wie zuvor beschrieben gewonnenen Transfektanten wurden zur Immunisierung von Balb/c-Mäusen (i.p.) verwendet. Nach vier Immunisierungen im Abstand von 14 Tagen erfolgte die Fusion von Milzzellen mit der Myelomlinie SP2/0.

Eine Immunisierung kann auch mit der humanen Neuroblastom-Zelllinie CHP-126 (DSMZ Nr. ACC 304) erfolgen, um Antikörper gegen humanes FZD-4, und mit der humanen Retinoblastom-Zelllinie (WERI-RB-1 (DSMZ Nr. Acc 90), um Antikörper gegen humanes FZD-7 oder FZD-9 bzw. FZD-6 zu erhalten. Diese Zellen zeigen eine hohe Expression der jeweiligen FZD-Rezeptoren. Eingesetzt werden jeweils 10⁷ Zellen.

Hybridomüberstände, die mit der Transfektante bzw. zuvor mit den CHP-126- oder WERI-RB-1-Zellen, nicht jedoch mit den Wildtyp- oder Kontrollzellen reagierten, wurden ausgewählt und die entsprechenden Hybridome kloniert. Positive Klone wurden selektiert.

Auf diese Art und Weise wurden vier Klone erhalten. Diese wurden mit CH3A4A7, der monoklonale Antikörper gegen FZD-4 sezerniert, W3D2B10, der monoklonale Antikörper gegen FZD-7 sezerniert, und W3C4E11 bezeichnet, der monoklonale Antikörper gegen FZD-9 bzw. FZD-6 sezerniert. Die Antikörper weisen folgende Isotypen auf: CH3A4A7 und W3D2B10 jeweils Maus IgG1, W3CH411 Maus IgM.

### Untersuchung der Reaktivität der Antikörper auf definierten Stammzellpopulationen

Die wie zuvor beschrieben gewonnenen Antikörper wurden hinsichtlich ihrer Reaktivität auf definierten Stammzellpopulationen getestet. Die Ergebnisse dieser Untersuchungen sind in der folgenden Tabelle 1 dargestellt.

**Tab. 1: Reaktivität der erfindungsgemäßen Antikörper mit verschiedenen Zelltypen. Bei den verschiedenen Zelltypen handelt es sich um Tumorzelllinien (*), Primärzellen (+), kultivierte Primärzellen (○), Leukämiezellen (⊙)**

| | Antikörper | | |
|---|---|---|---|
| Zelltyp | CH3A4A7 (anti-FZD-4-mab) | W3C4E11 (anti-FZD-9-mab) | W3D2B10 (anti-FZD-7-mab) |
| Calu-1* | nd | - | - |
| T-47D* | + | - | - |
| A172* | + | - | - |
| NCH-H69* | (+) | - | (+) |
| DU-4475* | - | - | - |
| JEG-3* | + | - | - |
| MCF-7* | + | - | - |
| Weri-Rb-1* | + | + | + |
| NT-2* | + | + | - |
| 293E* | - | - | - |
| CHP-126* | + | (+) | - |
| HELA* | - | - | - |
| SK-Lu-1* | (+) | - | - |
| PB⁺ | - | - | - |
| KM⁺ | | - | - |
| CD34 + KM⁺ | - | - | - |
| NPC⁺ | + | - | - |
| MSC^{○} | nd | - | - |
| HUVEC^{○} | - | - | - |
| EM-2^{⊙} | - | - | - |
| HL-60^{⊙} | - | - | - |
| KG-1^{⊙} | - | - | - |
| KG-1a^{⊙} | - | - | - |
| U266^{⊙} | - | - | + |
| Nalm-1^{⊙} | (+) | - | - |
| Reh^{⊙} | - | - | + |
| BV-173^{⊙} | nd | - | - |
| CML-T1^{⊙} | - | + | + |
| MO7-e^{⊙} | - | - | - |
| MOLM-1^{⊙} | - | - | - |
| HEL^{⊙} | - | - | - |
| KU-812^{⊙} | - | - | - |
| UT-7^{⊙} | - | - | - |
| MEG-O1 | - | - | - |
| TF-1^{⊙} | - | - | - |
| K562^{⊙} | - | - | - |
| U937^{⊙} | - | - | - |
| CCRF-CEM^{⊙} | - | - | - |

Informationen über die einzelnen Zelltypen sind im Internet unter http://www.dsmz.de zu finden. Der Inhalt der Beschreibungen der gelisteten Zelltypen ist durch Bezugnahme Bestandteil der vorliegenden Anmeldung. Hierbei steht NPC für neuronale Progenitorzellen, MSC für mesenchymale Stammzellen, KM für Knochenmark, CD34⁺KM für CD34-positive KM-Zellen (Stammzellen), PB für periphere Blutzellen und HUVEC für "human umbilical vein endothelial cells".

Dabei zeigte sich, dass eine Vielzahl von Tumorzellen FZD-Rezeptoren exprimieren und diese Zellen mit den bereitgestellten Antikörpern spezifisch selektiert und ggf. isoliert werden können.

Die dazugehörigen FACS-Ausdrucke sind auszugsweise in der Fig. 1 dargestellt. Die Teilabbildung (A) zeigt das Reaktionsprofil des Antikörpers CH3A4A7 in der Form von FACS-Histogrammen bzw. FACS-Ausdrucken gegenüber HEK-293-Wildtypzellen (HEK-293), gegenüber den Transfektanten (HEK-293/FZD-4), gegenüber neuronalen Progenitorzellen (NPC), gegenüber mobilisiertem peripheren Blut (mPB), gegenüber Knochenmarkszellen (BM) und gegenüber mesenchymalen Stammzellen (MSC). Gleichermaßen wurde unter Verwendung eines Anti-Flag-Antikörpers (a-Flag) verifiziert, dass das jeweilige Frizzled-Protein in der Membran exprimiert wird. In Teilabbildung (B) sind die entsprechenden Histogramme für den W3D2B10-Antikörper, und in der Teilabbildung (C) die entsprechenden Histogramme für den W3C4E11-Antikörper dargestellt. Die FACS-Analysen wurden gemäß Standardverfahren durchgeführt, wie dies bspw. in der DE 102 42 337 A1 beschrieben ist, deren Inhalt durch Bezugnahme Gegenstand der vorliegenden Anmeldung ist.

Dabei wurde insbesondere festgestellt, dass der monoklonale Antikörper, der von dem Klon CH3A4A7 sezerniert wird, hochspezifisch und selektiv nur solche Transfektanten erkennt, die mit dem FZD-4-Konstrukt transfiziert wurden [Fig. 1A (HEK-293/FZD4) und Daten nicht gezeigt]. Entsprechendes gilt hinsichtlich FZD-7 für den Antikörper, der von dem Klon W3D2B10 sezerniert wird. Letzterer erkennt hochspezifisch und selektiv nur solche HEK-293-Transfektanten, die mit dem FZD-7-Konstrukt transfiziert wurden [Fig. 1B (HEK-293/FDZ7) und Daten nicht gezeigt]. Ferner erkennt der monoklonale Antikörper, der von dem Klon W3C4E11 sezerniert wird, hochspezifisch und selektiv nur solche HEK-293-Transfektanten, die mit dem FZD-9-Konstrukt transfiziert wurden [Fig. 1C (HEK-293/FZD9) und Daten nicht gezeigt).

Die erfindungsgemäßen Antikörper sind deshalb hochselektiv und spezifisch.

### Verwendung der Antikörper

Die Verwendung der erfindungsgemäßen Antikörper erfolgt gemäß üblicher Praxis bspw. im Westernblot, der Immunpräzipitation oder anderen immunologischen Verfahren. Dazu werden die Antikörper in üblichen Puffern, wie z.B. Tris- oder HEPES-Puffern, in gewünschter Konzentration eingesetzt, die durch bspw. Titrationsexperimente leicht bestimmt werden kann. Nähere Informationen über die Verwendung der erfindungsgemäßen Antikörper finden sich in Standardwerken der Molekularbiologie, wie bspw. der Abhandlung von Ed Harlow und David Lane (1998), "Using Antibodies: A Laboratory Manual", Cold Spring Harbor Laboratory Press, deren Inhalt durch Inbezugnahme Bestandteil der vorliegenden Anmeldung ist.

Die Erfinder haben erstmals monoklonale Antikörper bereitgestellt, die hochspezifisch und hochselektiv die humane Variante des FZD-4-Rezeptors erkennen. Bei den bereitgestellten Antikörpern handelt es sich um äußerst wertvolle Tools, die sowohl in der Grundlagenforschung als auch in der Diagnostik und Pharmazeutik verwendet werden können.

## Patentansprüche

1. Monoklonaler Antikörper oder Fragment davon, das die Antigenbindungsfunktion des Antikörpers beibehält, **dadurch gekennzeichnet, dass** dieser bzw. dieses spezifisch oder/und selektiv an den humanen Frizzled-4 Rezeptor bindet und dass der monoklonale Antikörper von der Hybridomzelle produziert wird, die in der Kultur CH3A4A7 bei der DSMZ unter der Nummer DSM ACC 2667 hinterlegt wurde.

2. Monoklonaler Antikörper oder Fragment davon nach Anspruch 1, **dadurch gekennzeichnet, dass** an diesen bzw. dieses ein Marker oder/und ein therapeutischer wirkstoff gekoppelt ist.

3. Hybridomzelle, **dadurch gekennzeichnet, dass** diese den Antikörper nach einem Anspruch 1 produziert.

4. Verfahren zur Detektion oder/und Isolierung des humanen FZD-4 Rezeptors oder Fragmenten davon in einer biologischen Probe, das folgende Schritte aufweist:
(1) In-Kontakt-bringen der biologischen Probe mit einem Antikörper;
(2) Feststellung, ob eine spezifische oder/und selektive Bindung an den Antikörper stattgefunden hat, und
(3.1) Korrelation einer positiven Feststellung in Schritt (2) mit der Detektion von humanem FZD-4 Rezeptor, oder/und
(3.2) Isolierung des Antigens bei einer positiven Fest-
stellung in Schritt (2),
**dadurch gekennzeichnet, dass** als Antikörper der Antikörper oder das Fragment davon nach einem der Ansprüche 1 oder 2 verwendet wird.

5. In-vitro-Verfahren zur Identifizierung von biologischem Material hinsichtlich des Vorhandenseins oder/und der Ex- pression des humanen FZD-4 Rezeptors oder Fragmenten davon und ggf. Isolierung eines solchen Materials, das folgende Schritte aufweist:
(1) In-Kontakt-bringen des biologischen Materials mit einem Antikörper;
(2) Feststellung, ob eine spezifische oder/und selektive Bindung an den Antikörper stattgefunden hat, und
(3.1) Korrelation einer positiven Feststellung in Schritt (2) mit der Identifizierung des Vorhandenseins oder/und der Expression von humanem FZD-4 Rezeptor oder Homologen oder Fragmenten davon in bzw. auf dem biologischen Material, und ggf.
(3.2) Isolierung des Bindepartners bei einer positiven
Feststellung in Schritt (2),
**dadurch gekennzeichnet, dass** als Antikörper der Antikörper oder das Fragment davon nach einem der Ansprüche 1 oder 2 verwendet wird.

6. Verwendung des Antikörpers nach einem der Ansprüche 1 oder 2 zur spezifischen oder/und selektiven in vitro Detektion oder/und Isolierung des humanen FZD-4 Rezeptors.

7. Verwendung nach Anspruch 6 zur in vitro Analyse der Karzinogenese oder/und der Differenzierung und Organogenese embryonaler Gewebe oder/und der Oogenese und Keimblattentwicklung oder/und der Selbsterneuerung von Stammzellen.

8. Zusammensetzung, die den monoklonalen Antikörper oder Fragmente davon nach einem der Ansprüche 1 oder 2 aufweist.

9. Zusammensetzung nach Anspruch 8, **dadurch gekennzeichnet, dass** diese eine pharmazeutische Zusammensetzung ist und einen pharmazeutisch akzeptablen Träger sowie ggf. weitere Zusätze aufweist.

10. Kit, das den monoklonalen Antikörper oder Fragmente davon nach einem der Ansprüche 1 oder 2 aufweist.

## Claims

1. A monoclonal antibody or a fragment thereof which holds the antigen-binding function of the antibody, **characterized in that** it specifically and/or selectively binds to the human frizzled-4 receptor and that the monoclonal antibody is produced by the hybridoma cell, which was deposited in culture CH3A4A7 at the DSMZ under number DSM ACC 2667.

2. Monoclonal antibody or fragment thereof as claimed in claim 1, **characterized in that** a marker and/or a therapeutic active substance is coupled to it.

3. Hybridoma cell, **characterized in that** it produces the antibody as claimed in claim 1.

4. Method for detecting and/or isolating human FSZ-4 receptors or fragments thereof in a biological sample, comprising the following steps:
(1) Contacting said biological sample with an antibody;
(2) determination whether specific and/or selective binding to said antibody has taken place, and
(3.1) correlation of a positive result in step (2) with the detection of a human FZD-4 receptor, or/and
(3.2) isolation of the antigen in the case of a positive result in step (2), **characterized in that** the antibody or the fragment thereof as claimed in claims 1 or 2 is used as antibody.

5. In-vitro-method for identification of biological material with respect to the presence or/and expression of the human FZD-4 receptors or fragments thereof and optionally isolation of said material, comprising the following steps:
(1) Contacting said biological material with an antibody;
(2) determination whether specific and/or selective binding to said antibody has taken place, and
(3.1) correlation of a positive finding in step (2) with the identification of the presence and/or expression of human FZD-4 receptor or homologues or fragments thereof in or on the biological material, and optionally
(3.2) isolation of the binding partner if the determination in step (2) is posi-
tive,
**characterized in that** the antibody or a fragment thereof as claimed in one of claims 1 or 2 is used as said antibody.

6. Use of an antibody as claimed in claim 1 or 2 for specific and/or selective in vitro detection or/and isolation of the human FZD-4 receptor.

7. The use as claimed in claim 6 for in vitro analysis of the carcinogenesis and/or the differentiation and organogenesis of embryonic tissue and/or the oogenesis and development of the germ layer and/or the auto regeneration of stem cells.

8. Composition comprising the monoclonal antibody or fragments thereof as claimed in claims 1 or 2.

9. Composition as claimed in claim 8, **characterized in that** it is a pharmaceutical composition and comprises a pharmaceutically acceptable excipient and optionally other additives.

10. Kit comprising the monoclonal antibody or fragments thereof as claimed in one of claims 1 or 2.

## Revendications

1. Anticorps monoclonal ou fragment de celui-ci qui conserve la fonction de liaison à l'antigène de l'anticorps, **caractérisés en ce que** l'un ou l'autre se lie spécifiquement et/ou sélectivement au récepteur Frizzled-4 humain et **en ce que** l'anticorps monoclonal est produit par la cellule d'hybridome qui a été déposée dans la culture CH3A4A7 auprès de la DSMZ sous le numéro DSM ACC 2667.

2. Anticorps monoclonal ou fragment de celui-ci selon la revendication 1, **caractérisés en ce qu'**un marqueur et/ou une substance active thérapeutique sont couplés à l'un ou à l'autre.

3. Cellule d'hybridome, **caractérisée en ce qu'**elle produit un anticorps selon la revendication 1.

4. Procédé pour la détection et/ou l'isolement du récepteur FZD---4 humain ou de fragments de ce récepteur dans un échantillon biologique, comprenant les étapes suivantes consistant à :
(1) mettre en contact l'échantillon biologique avec un anticorps;
(2) déterminer si une liaison spécifique et/ou sélective à l'anticorps a eu lieu, et
(3.1) établir une corrélation entre une détermination positive à l'étape (2) et la détection du récepteur FZD-4 humain et/ou
(3.2) isoler l'antigène en cas de détermination positive à l'étape (2), **caractérisé en ce que** l'on utilise comme anticorps l'anticorps ou le fragment de celui-ci selon l'une des revendications 1 ou 2.

5. Procédé *in vitro* pour l'identification de matériel biologique au vu de la présence et/ou de l'expression du récepteur FZD-4 humain ou de fragments de celui-ci et le cas échéant l'isolement d'un tel matériel, comprenant les étapes suivantes consistant à :
(1) mettre en contact le matériel biologique avec un anticorps;
(2) déterminer si une liaison spécifique et/ou sélective à l'anticorps a eu lieu, et
(3.1) établir une corrélation entre une détermination positive à l'étude (2) et l'identification de la présence et/ou de l'expression du récepteur FZD-4 humain ou d'homologues ou de fragments de celui-ci dans ou sur le matériel biologique et, le cas échéant,
(3.2) isoler le partenaire de liaison par une détermination positive à l'étape (2),
**caractérisé en ce que** l'on utilise comme anticorps l'anticorps ou le fragment de celui-ci selon l'une des revendications 1 ou 2.

6. Utilisation de l'anticorps selon l'une des revendications 1 ou 2 pour la détection et/ou l'isolement *in vitro* spécifiques et/ou sélectifs du récepteur FZD-4 humain.

7. Utilisation selon la revendication 6 pour l'analyse *in vitro* de la carcinogenèse et/ou de la différenciation et de l'organogenèse de tissus embryonnaires et/ou de l'ovogenèse et du développement du feuillet germinatif et/ou de l'autorenouvellement de cellules souches.

8. Composition qui contient l'anticorps monoclonal ou le fragment de celui-ci selon l'une des revendications 1 ou 2.

9. Composition selon la revendication 8, **caractérisée en ce qu'**elle est une composition pharmaceutique et qu'elle contient un véhicule pharmaceutiquement acceptable ainsi que, le cas échéant, d'autres additifs.

10. Kit contenant l'anticorps monoclonal ou le fragment de celui-ci selon l'une des revendications 1 ou 2.
